# EUROPEAN PATENT APPLICATION

(11) **EP 3 147 372 A2**
(43) Date of publication of application: **29.03.2017**
(21) Application number: 16185746.1
(22) Date of filing: 21.11.2012
(51) Int. Cl.: C12Q 1/68, G01N 33/543

(54) **VERSATILE AND SENSITIVE BIOSENSOR**

(30) Priority: 23.11.2011 US 201161563130 P
(62) Divisional of application: 12851735.6
(71) Applicant: THE GOVERNING COUNCIL OF THE UNIVERSITY OF TORONTO, Toronto, Ontario M5S 1A1 (CA)
(72) Inventor: KELLEY, Shana, O., Toronto, Ontario M4V 2R4 (CA); ZARAGOZA, Alexandre, Toronto, Ontario M6N 3A6 (CA); SARGENT, Edward, Hartley, Toronto, M5S 3G4 (CA); DAS, Jagotamoy, Toronto, Ontario M4Y 1R5 (CA); CEDERQUIST, Kristin, Lansing, Michigan 48917 (US)
(74) Representative: Cooley (UK) LLP

(57) **Abstract**

Contemplated methods and devices comprise use of a charged probe and a pseudoligand in the electrochemical detection of a wide range of analytes, including nucleic acids, proteins and small molecules. In certain embodiments, the pseudoligand forms a complex with the probe that has a reduced charge magnitude compared to the probe itself, and is displaced from the probe when the complex is exposed to the analyte.

## Description

### Related Application

This claims the benefit of priority to U.S. Provisional Patent Application No. 61/563,130, filed November 23, 2011 which application is hereby incorporated herein by reference in its entirety.

### Field of the Invention

The field of the invention is analytical devices for characterizing or detecting a wide range of analytes, including nucleic acids, proteins, and small molecules.

### Background

The development of universal sensors that can detect a broad range of different molecular targets is highly desirable. For example, such versatile platforms have the potential to provide a single solution for tests that are run using different types of instrumentation. However, to date, very few universal detection systems have been developed and none have sufficient sensitivity for direct sample analysis or clinical use. Furthermore, detection methods that are rapid and more sensitive than those currently available will fulfill unmet needs in screening for drugs of abuse, medical diagnosis, point-of-care testing, and environmental monitoring.

Electrochemical detection is an attractive modality for such universal sensors, as it does not rely on complex and relatively fragile optical systems and the sensor surface may be fabricated as a compact and relatively inexpensive microchip containing an array of sensors with different specificities that may be read essentially simultaneously. Sensing approaches that report on changes in the electrostatics of a sensor-immobilized monolayer have been developed with a variety of readout strategies, including field-effect transistors (Tian, B. et al (2010) Science 13:830-834). microcantilevers (Wu, G., et al. (2001) Nat. Biotechnol. 19:856-860), and electrochemical sensors (Drummond, T. G.; Hill, M. G.; and Barton, J. K. (2008) Nat. Biotechnol. 21:1192-1199). However, an effective method that can sensitively detect a wide variety of analytes has remained elusive. Electrochemical signaling methods have attracted particular attention for fast, sensitive, portable, and cost-effective detection. One electrochemical system has shown promise for versatile detection, but with a limited sensitivity towards nucleic acids analytes that require complex and time consuming enzymatic amplification of target sequences prior to detection (Lai, R. L. et al (2006) Proc. Natl. Acad. Sci. 103:4017-4021).

### Summary

The devices and methods described herein provide a new approach to electrochemical detection that affords excellent sensitivity to a wide range of analytes, including nucleic acids, proteins, and small molecules. In certain embodiments, a probe sequence or probe aptamer is immobilized on a sensor surface that detects local charge. This probe sequence or probe aptamer is exposed to a pseudoligand, or neutralizer, which complexes with and has a charge opposed to that of the probe sequence or probe aptamer, thereby reducing the magnitude of the total or overall charge that is present in the local environment of the probe. In certain embodiments, a sample that may contain an analyte is contacted with the probe. The analyte of interest, if present in the sample, forms a complex with the probe sequence or probe aptamer. The formation of the complex displaces the neutralizer, thereby changing the charge state of the local environment of the probe by, for example, generating a higher charge density near the test surface that is subsequently detected. The neutralizer may contain one or more sequence mismatches in order to improve the efficiency of displacement from the probe sequence or probe aptamer by the analyte.

Any suitable sensor surface may be used. In certain embodiments, the sensor surface provides a response that is charge dependent. In certain embodiments, the sensor surface is a nanostructured electrochemical detection electrode. In certain embodiments, the sensor surface is a field-effect transistor, a microcantilever, or an electrochemical sensor. In certain embodiments, at least two sensor surfaces with different probes affixed are used, which are capable of forming complexes with different analytes.

The analyte can be any substance or chemical of interest in an analytic procedures, including without limitation nucleic acids, proteins, and small molecules. In one embodiment, the analyte of interest may be a small molecule, including but not limited to a therapeutic drug, a drug of abuse, environmental pollutant, and free nucleotides. In such an embodiment, the probe may be an aptamer configured to bind the small molecule, and can include a neutralizer that complexes with the probe and is displaced by the small molecule.

In certain embodiments, the relative stabilities between the probe and pseudoligand, the probe and the analyte, and the analyte and the pseudoligand can be modified by manipulating the temperature. In certain embodiments, the relative stabilities between the probe and pseudoligand, the probe and the analyte, and the analyte and the pseudoligand can be modified by the composition of a buffer solution in which the complexes form.

In certain embodiments, the analyte of interest may be a target nucleic acid, including but not limited to DNA, RNA, and peptide nucleic acid (PNA). In such embodiments the probe may be a nucleic acid sequence that is at least partially complementary to the analyte nucleic acid, and can include a neutralizer that complexes with the probe sequence and is displaced by the target nucleic acid. In certain embodiments, the probe may comprise a nucleic acid, such as DNA or PNA. In certain embodiments, the pseudoligand may comprise a PNA.

In certain embodiments, the analyte of interest may be a protein or protein fragment. In such embodiments the probe may be an aptamer configured to bind to the protein or protein fragment, and can include a neutralizer that complexes with the probe aptamer and is displaced by the protein or protein fragment. In certain embodiments, the analyte of interest may be an uncharged molecule. In certain embodiments, the analyte is a small molecule with a molecular weight of less than about 500 daltons.

In certain embodiments, the analyte of interest binds to the neutralizer with high affinity. In such embodiments, formation of a complex between the neutralizer and the analyte frees the neutralizer from the probe, thereby causing charge near the sensor surface to increase in magnitude. In such embodiments, the neutralizer may incorporate one or more base pair mismatches in order to reduce its affinity for the probe.

### Brief Description of the Drawings

The foregoing and other objects and advantages will be apparent upon consideration of the following detailed description, taken in conjunction with the accompanying drawings, in which like reference characters refer to like parts throughout.
FIG. 1A illustrates the prior art method for electrostatic detection using an electronegative probe on a sensor surface.
FIG. 1B illustrates an embodiment wherein a large change in the charge near the detection surface is generated when an analyte displaces a pseudoligand from a charged probe immobilized on the detection surface.
FIG. 2 shows an illustrative process for detecting an analyte in accordance with certain embodiments.
FIG. 3A illustrates an embodiment wherein charged reporter ions are drawn towards the probe on release of the neutralizer and binding of a target.
FIG. 3B shows an exemplary sensor chip containing multiple nanostructured detection electrodes.
FIG. 3C shows an exemplary nanostructured detection electrode.
FIG. 4 shows an exemplary system for detecting an analyte in accordance with certain embodiments.
FIG. 5 shows a list of illustrative probe and neutralizer sequences.
FIG. 6A shows an illustrative ATP biosensor, wherein displacement of a neutralizer from a DNA probe aptamer on a detection electrode occurs when ATP complexes with a probe aptamer.
FIG. 6B shows illustrative differential pulse voltammograms for an ATP biosensor in the absence of neutralizer (aptamer only), after neutralizer has complexed with the probe aptamer (+ neutralizer), and following displacement of the neutralizer from the probe aptamer by ATP (+ ATP).
FIG. 6C shows the relationship between signal strength and ATP concentration for an illustrative ATP biosensor.
FIG. 6D shows the time dependence of the signal change observed from an illustrative ATP biosensor on the addition of ATP.
FIG. 7A shows an illustrative cocaine biosensor, wherein displacement of a neutralizer from a probe aptamer on a detection electrode occurs when cocaine complexes with the probe aptamer.
FIG. 7B shows illustrative differential pulse voltammograms for a cocaine biosensor in the presence of cocaine (+ cocaine) and in the absence of cocaine (- cocaine).
FIG. 7C shows the relationship between signal strength and cocaine concentration for an illustrative cocaine biosensor.
FIG. 8A shows an illustrative nucleic acid biosensor, wherein displacement of a neutralizer from a probe sequence immobilized on a detection electrode occurs when a target nucleic acid complexes with the probe sequence.
FIG. 8B shows differential pulse voltammograms for a DNA biosensor in the absence of neutralizer (probe only), after neutralizer has complexed with the probe sequence (+ neutralizer), and following displacement of the neutralizer from the probe sequence by 1pM of a complementary 20mer DNA target (+ DNA target).
FIG. 8C shows the relationship between signal strength and complementary 20mer DNA target concentration for a DNA biosensor.
FIG. 8D shows the relationship between signal strength and total *E. coli* RNA concentration for a biosensor utilizing a probe complementary to *rpoB*.
FIG. 8E shows the relationship between signal strength and lysates obtained from different concentrations *of E. coli* for an illustrative biosensor utilizing a probe complementary to *rpoB*.
FIG. 9A shows an illustrative protein biosensor, wherein displacement of a neutralizer from a probe aptamer immobilized on a detection electrode occurs when a protein (in this instance thrombin) complexes with the probe aptamer.
FIG. 9B shows differential pulse voltammograms for an illustrative thrombin biosensor in the absence of neutralizer (aptamer only), after neutralizer has complexed with the probe aptamer (+ neutralizer), and following displacement of the neutralizer from the probe aptamer by 100 pM thrombin (+ thrombin).
FIG. 9C shows differential pulse voltammograms for a thrombin biosensor in the presence of the nonspecific blocking protein bovine serum albumin (+ BSA) and absence of bovine serum albumin (- BSA).
FIG. 9D shows the relationship between signal strength and thrombin concentration for a thrombin biosensor. The horizontal dashed line shows the signal generated by 100nM bovine serum albumin, a nonspecific protein.
FIG. 10 shows an illustrative nucleic acid biosensor, wherein displacement of a neutralizer from a probe sequence immobilized on a detection electrode occurs when a target nucleic acid complexes with the neutralizer.

### Detailed Description

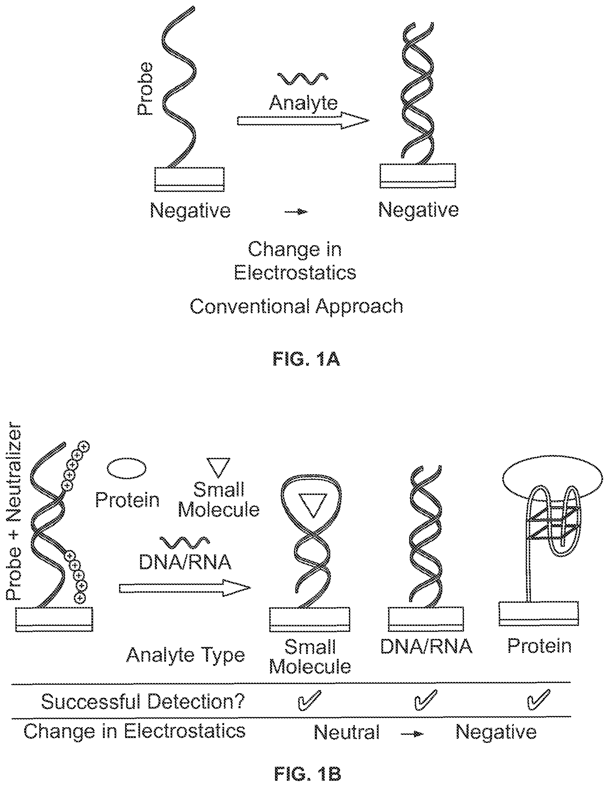

The principles underlying prior art assays are illustrated in FIG. 1A. In prior art assays, as illustrated in FIG. 1A, a non-complexed probe molecule is affixed to the surface of a detection electrode. As a result, the charge of the sensor is determined solely by the probe molecule prior to introduction of the analyte, the analyte being a ligand that binds to and forms a stable complex with the probe molecule. Following binding by the analyte, the charge state at the electrode surface is changed by the charge of the analyte. This approach leads to significant limitations in prior art charge-based sensing methods. First, the background signal may be large due to the inherent charge of the probe molecule, which is often an electronegative nucleic acid. As a result the ratio of the signal resulting from analyte complexing with the probe to the background signal may be small if the charge of the probe is very large relative to the charge of the analyte, resulting in limited assay sensitivity. As a result, such assays often require tedious, expensive, and error prone amplification of the analyte (by methods such as PCR) prior to analysis. Uncharged analytes that do not produce a significant change in the charge at the electrode surface when they complex with the probe, particularly low molecular weight analytes, may be undetectable. Finally, the detection signal is often a reduction in charge magnitude at the electrode surface as a result of complex formation between the affixed probe and the analyte. This leads to a "signal-off" assay structure in which presence of the analyte is indicated by a lack of signal, a configuration that often results in a high rate of false-positive determinations. Prior art methods of sensing are, essentially, dependent upon the nature of the analyte for their signal amplitude, their signal-to-background ratio, and their sign of signal change. Unfortunately, the choice of analyte is a not a variable that the assay designer can change, but rather is a requirement of the test.

The embodiments illustrated in FIG. 1B introduce a new freedom in the design of the electrostatic character of the sensor surface. A probe molecule is affixed to the surface of an electrode and is complexed with a neutralizer, the neutralizer being a pseudoligand that has an affinity for the probe and neutralizes the probe's charge on complex formation. In certain embodiments, a device may be supplied to the user with the neutralizer already complexed to the probe. In certain embodiments, the user may apply the neutralizer to a probe-containing element such as a detection electrode prior to the addition of analyte. In yet another embodiment the user may apply the neutralizer to a probe-containing element essentially simultaneously with the addition of the analyte. The neutralizer acts as a pseudoligand that forms a reversible complex with the probe and can be displaced by a ligand or analyte that forms a complex with the probe. Towards this end the neutralizer may incorporate base pair mismatches with the probe such that the analyte of interest binds the probe more strongly, rapidly and/or robustly, leading to displacement of the neutralizer. The affinity of the neutralizer for the probe may also be modified using temperature changes or changes in buffer composition. Such changes in buffer composition include, but are not limited to, changes in ionic strength, presence or absence of multivalent cations, presence or absence of organic solvents, presence or absence of chaotropes, and presence or absence of hydrophilic polymers.

The neutralizer may be any molecule that forms a complex with the probe molecule. In certain embodiments, such a complex has a reduced charge magnitude compared to the affixed probe such that the neutralizer is displaced from such a complex on the addition of target analyte. The neutralizer may be a nucleic acid analog that incorporates a neutral or a positively charged backbone structure. The neutralizer may also be a nucleic acid analog that incorporates a negatively charged backbone structure but has a net positive charge. In certain embodiments, the neutralizer is a conjugate of peptide nucleic acid and cationic amino acids that specifically bind to an electronegative probe so that the charge of the neutralizer-probe complex is less electronegative than that of the probe alone. In other embodiments the neutralizer may incorporate morpholino nucleic acid analogs or methylphosphonate nucleic acid analogs.

The use of a displaceable pseudoligand allows various embodiments to overcome the limitations of traditional charge-sensing assays. In certain embodiments, the background signal in the assay is suppressed through charge compensation that is engineered by the assay designer, thereby enhancing the signal detection. In such embodiments, the signal changes that correspond to the presence of an analyte are determined not only by the molecular charge of the analyte ligand, but also by the inherent charge of the probe molecule, which is unmasked upon release of the neutralizer. This permits the detection of analytes that do not produce significant changes in the charge of the probe upon complex formation, permitting the use of assays with a range of low molecular weight analytes that could not previously be addressed by electrochemical detection. Such low molecular weight molecules typically have a molecular weight of less than about 500 daltons, and may include but are not limited to nucleotides and nucleotide analogs, drugs of abuse, therapeutic drugs, and environmental contaminants.

In certain embodiments, the suppression of background signal also greatly improves the analyte-specific signal to background signal ratio. Surprisingly, this reduction in the analyte-specific signal to background signal permits direct detection of nucleic acid analytes, removing the need for expensive and time-consuming PCR amplification of samples prior to characterization or detection.

In certain embodiments, the sign and amplitude of signal is determined not only by the charge of the analyte but also by that of the probe. This permits design of a "signal-on" assay in which presence of the analyte is indicated by a magnitude increase in the measured signal. Such signal-on assays generally show a low rate of false positive results relative to assays with a signal-off structure.

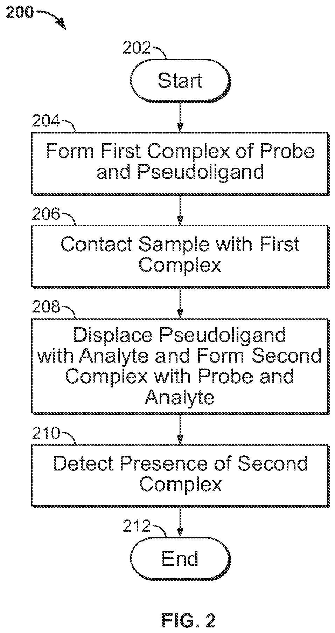

FIG. 2 shows an illustrative process 200 for detecting an analyte in accordance with certain embodiments. The process begins at step 202. At step 204, a reversible first complex is formed between a probe affixed to a sensor surface and a pseudoligand. The pseudoligand may be partially complementary to the probe, and has a charge that is opposed to that of the probe. At step 206, a sample that may contain the analyte is contacted with the first complex at the sensor surface. If the analyte is present in the sample, at step 208, the pseudoligand is displaced by the analyte and a second complex is formed. In certain embodiments, the pseudoligand is displaced by the analyte and a second complex is formed between the analyte and the probe. In certain embodiments, the pseudoligand is displaced by the analyte and a second complex is formed between the analyte and the pseudoligand. At step 210, the presence of the second complex is detected, which indicates that the analyte is present in the sample. The process ends at step 212. It is understood that the steps of process 200 are merely illustrative and that certain steps may be performed simultaneously and/or performed in another suitable order without departing from the scope of the invention. In certain embodiments, process 200 also includes a step of communicating the result of the detection (not shown), for example, by displaying an indicator (e.g., displaying a text, symbol, or color-coded indicator) to a user of the process. In certain embodiments, the step of communicating the results includes storing the result in a local or remote memory associated with the process 200, or by sending a message to a user of the process 200.

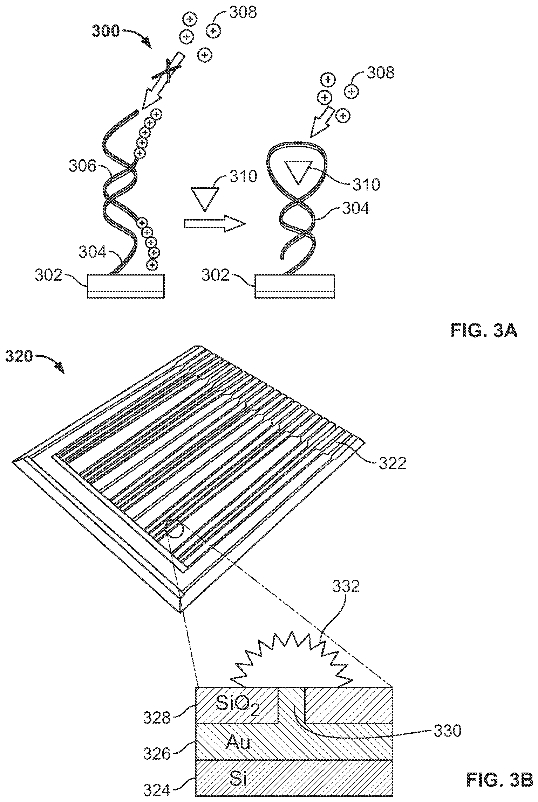

FIG. 3A shows an illustrative embodiment which was tested using an electrocatalytic reporter system that provides a signal proportional to the magnitude of the charge change at electrode surfaces. To measure the change of charge at the sensor surface, a [Ru(NH₃)₆]³⁺/[Fe(CN)₆]³⁻ catalytic reporter system 300 was used to generate a signal that can be monitored by differential pulse voltammetry (DPV) in the presence of, for example, a low molecular weight molecule 310 such as a nucleotide and nucleotide analog, a drug of abuse, a therapeutic drug, and an environmental contaminant. In this illustrative system, the primary electron acceptor 308, which may be any suitable electron acceptor such as [Ru(NH₃)₆]³⁺, is electrostatically attracted to the electrode surface 302 in proportion to the amount of phosphate-bearing nucleic acid 304. When [Fe(CN)₆]³⁻ is used during electrochemical readout, the Ru(III) is chemically regenerated by [Fe(CN)₆]³⁻ forming a redox cycle, which amplifies the signal significantly. This illustrative embodiment is free of covalent labels and does not require preprocessing of the samples. High catalytic currents would be expected when only DNA aptamer probes 304 are immobilized on the sensors 302 due to electrostatic affinity of Ru(III) for the phosphate groups of the DNA backbone, however in the tested assays these currents would be strongly attenuated in the presence of the neutralizer 306. This reporter system may be used with nanostructured microelectrodes that can be fabricated on the surface of a chip.

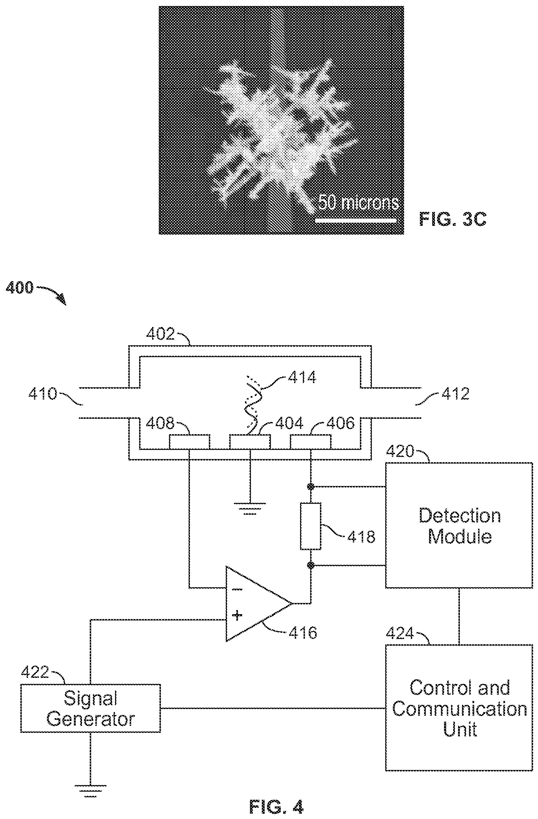

FIGS. 3B and 3C show an illustrative sensor used in an example embodiment. To fabricate the sensor, photolithographic patterning was used to produce a microelectronic chip 320 with an array of sensors 322. The chips used in this study possessed twenty sensors. With the use of a silicon wafer 324 coated with a gold (Au) layer 326 and a SiO₂ layer 328 as a base, contact pads and leads were patterned onto individual chips. An overlayer of Si₃N₄ was then used to passivate the surface of the chip. To provide a template for the growth of electrodeposited sensors, photolithography was then used to open 5 µm apertures 330 in the Si₃N₄. Gold electrodeposition was then employed to grow fractal microstructures 332, the size and morphology of which can be modulated by deposition time, potential, Au concentration, supporting electrolyte, and overcoating protocol by methods known in the art. As nanostructures increase the sensitivity of the assay significantly, Au structures were coated with a thin layer of Pd to form finely nanostructured sensors (FIG. 3C). It is understood that the materials, dimensions, and processes used to generate the sensors are merely illustrative and that other suitable materials, processes, or dimensions may be used without departing from the scope of the disclosure.

In certain embodiments, chips were fabricated using several inch silicon wafers that were passivated using a thick layer of thermally grown silicon dioxide. A 25 nm Ti was then deposited. A 350 nm gold layer was subsequently deposited on the chip using electron-beam-assisted gold evaporation, and patterned using standard photolithography and a lift-off process. A 5 nm Ti layer was then deposited. A 500 nm layer of insulating Si₃N₄ was deposited using chemical vapor deposition. 5 mm apertures were then imprinted on the electrodes using standard photolithography, and 0.4 mm × 2 mm bond pads were exposed using standard photolithography.

To fabricate the assay test sites in certain embodiments, chips were cleaned by sonication in acetone for 5 min, rinsed with isopropyl alcohol and deionized (DI) water, and dried with a flow of nitrogen. Electrodeposition was performed at room temperature; 5 µm apertures on the fabricated electrodes were used as the working electrode and were contacted using the exposed bond pads. Au (gold) sensors were made using a deposition solution containing 50 mM solutions of HAuCl₄ and 0.5 M HCl. 100 µm and 20 µm Au structures were formed using DC potential amperometry at 0 mV for 100 seconds and 0 mV for 20 seconds respectively. After washing with DI water and drying, the Au sensors were coated with Pd to form nanostructures by replating in a solution of 5 mM H₂PdCl₄ and 0.5 M HClO₄ at -250 mV for 10 seconds (for 100 micron structure) and for 5 seconds (for 20 micron structure).

In certain embodiments, an exemplary protocol for preparing the assays was used. In this protocol, thiolated aptamers and thiolated DNA probes were deprotected using dithiothreitol (DTT) followed by purification with HPLC. HPLC-purified probes were subsequently lyophilized and stored at -20 °C. Phosphate buffer solution (25 mM, pH 7) containing 5 µM thiolated probe, 25 mM NaCl, and 50 mM MgCl₂ was incubated with sensors for 1 hour in a dark humidity chamber at room temperature to immobilize the probe on the test surface. The chip was then washed twice for 5 minutes with phosphate buffer solution (25 mM) containing 25 mM NaCl. Sensors were then incubated with a phosphate buffer solution (25 mM) containing 10 µM neutralizer and 25 mM NaCl for 30 minutes at room temperature, followed by washing three times for 5 minutes with the same buffer. For the purposes of demonstrating detection, the chips were then treated with different analytes followed by washing.

In certain embodiments, electrochemical experiments were carried out using a Bioanalytical Systems (West Lafayette, Indiana) Epsilon potentiostat with a three-electrode system featuring a Ag/AgCl reference electrode and a platinum wire auxiliary electrode. Electrochemical signals were measured in a 25 mM phosphate buffer solution (pH 7) containing 25 mM NaCl, 10 µM [Ru(NH₃)₆]Cl₃, and 4 mM K₃[Fe(CN)₆]. Differential pulse voltammetry (DPV) signals were obtained with a potential step of 5 mV, pulse amplitude of 50 mV, pulse width of 50 msec, and a pulse period of 100 msec. Signal changes corresponding to replacement of the neutralizer by specific target were calculated with background-subtracted currents: ΔI% = (I_{after} - I_{befοre})/I_{befοre} × 100 (where I_{after} = current after replacement of neutralizer, I_{before} = current before replacement of neutralizer). In these illustrative embodiments, scanning electron microscope images were obtained using an Aspex (Delmont, Pennsylvania) 3025 SEM.

FIG. 4 shows an exemplary system for detecting an analyte in accordance with certain embodiments. The detection system 400 has a detection chamber 402 that includes one or more electrodes. In FIG. 4, the detection chamber includes a working electrode 404, a counterelectrode 406, and a reference electrode 408. However, any suitable number or types of electrodes may be used. The detection chamber 402 also has an inlet 410 for flowing in a sample for contacting with the working electrode 404, and outlet 412 for flowing out the sample. If the sample contains the analyte of interest, the analyte may form a probe-analyte complex 414 on the surface of the working electrode 404. In certain embodiments, once a sample enters the detection chamber 402 through the inlet 410, a certain amount of time may be allotted to facilitate formation of the probe-analyte complex 414. In certain embodiments, a sample containing the analyte may flow out through the outlet after enough time has been allotted for the probe-analyte complex 414 to form. A washing solution may subsequently flow in through the sample chamber 402 to remove undesirable materials that may be present in the sample.

The detection system 400 shown in FIG. 4 incorporates a illustrative three-electrode potentiostat configuration, however it is to be understood that any suitable configuration of components could be used. The counterelectrode 406 is connected to resistor 418, which is in turn connected to the output of control amplifier 416. A detection module 420 is connected across the resistor 418 to provide a current measurement. The detection module 420 may be configured to provide real-time current measurement in response to any input waveform. The reference electrode 408 is connected to the inverting terminal of control amplifier 416. A signal generator 422 is connected to the noninverting terminal of the control amplifier 416. This configuration maintains constant potential at the working electrode while allowing for accurate measurements of the current. In certain embodiments, the detection chamber 402 may contain a plurality of electrodes for detecting multiple analytes. For example, the detection chamber 402 may include multiple working electrodes each with a different type of probe affixed for complexing with different targets present in the sample. In certain embodiments, the detection system 400 may be configured to individually address the working electrodes one at a time while utilizing a common counterelectrode and reference electrode.

A control and communication unit 424 is operably coupled to the detection module 420 and the signal generator 422. The control and communication unit 424 may synchronize the input waveforms and output measurements, and may receive and store the input and output in a memory. In certain embodiments, the control and communication unit 424 may be a separate unit that interfaces with the detection system 400. For example, the detection system 400 may be a disposable cartridge with a plurality of input and output terminals that can connect to an external control and communication unit 424. In certain embodiments, the control and communication unit may be operably coupled to a display unit that displays the output as a function of input. In certain embodiments, the control and communication unit 424 may transmit the input and output information to a remote destination for storage and display. For example, the control and communication unit 424 could be a mobile device or capable of being interfaced with a mobile device. In certain embodiments, the control and communication unit 424 could provide power to the detection system 400. The system 400 maybe powered using any suitable power source, including a battery or a plugged-in AC power source.

In certain embodiments, a detection system may be provided as an assay composition for use in drug screening. The assay composition may have a reversible first complex comprising a probe molecule affixed to a sensor surface that forms a complex with a complementary or partially-complementary pseudoligand. The probe may have an affinity for a drug that is greater than that for the pseudoligand. Consequently, the pseudoligand may be displaced by the drug, forming a second complex. The first and second complexes may have first and second charge states, respectively. In certain embodiments, the detection system may be provided as a kit, which includes a device with a sensor surface and a probe affixed to the sensor surface. The kit may also have a pseudoligand capable of forming a reversible complex with the pseudoligand. The pseudoligand in the kit may be already complexed with the probe, or may be separately included with the kit for later complexation.

In various embodiments, the neutralizers were synthesized using a solid phase synthesis approach on a Prelude automated peptide synthesizer (Protein Technologies, Inc.; Tucson, Arizona). In these embodiments, synthesis products were confirmed by mass spectroscopy.

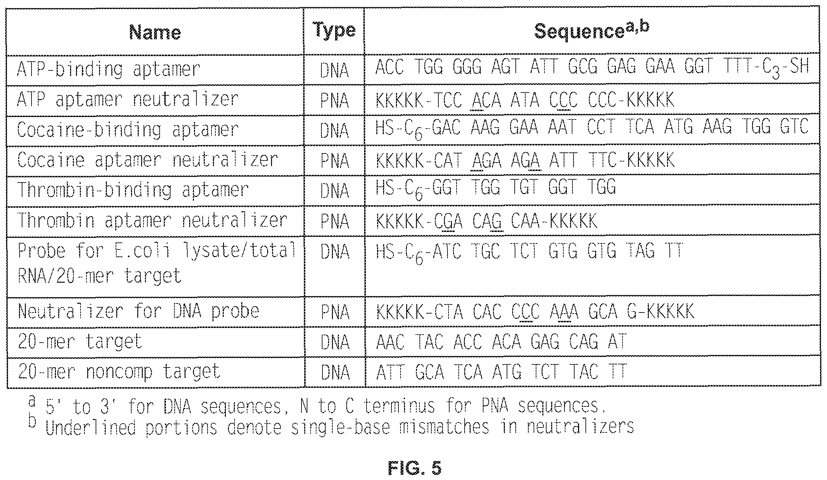

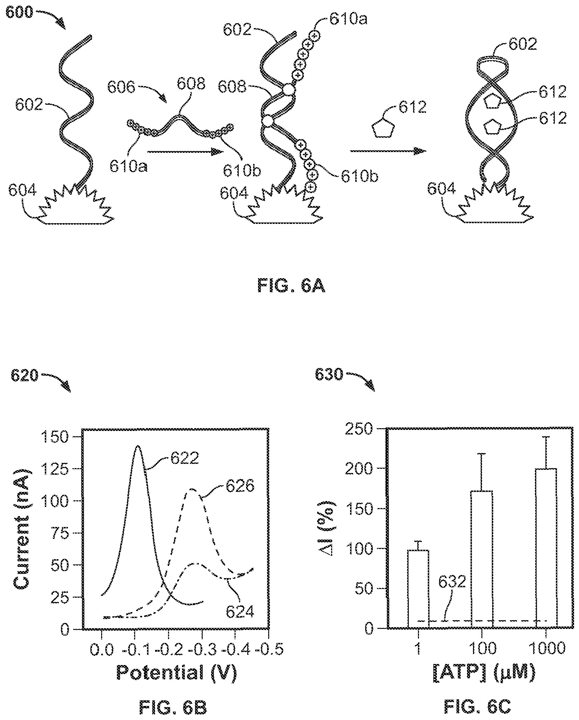

In certain embodiments, to examine the ability of the assay to detect small molecules, ATP was selected as a model analyte or binding ligand. Illustrative ATP probe and aptamer sequences are shown in FIG. 5. An illustrative configuration of the neutralizer assay 600 for ATP detection is shown in FIG. 6A. In these embodiments, thiolated ATP-binding aptamers 602 are first immobilized onto sensors 604 with Pd on their surfaces, and a partially complementary neutralizer 606 is then introduced. The PNA portion 608 of the neutralizer 606 is primarily complementary to the aptamer 602. However, two mismatches 610 were introduced to permit facile release of the neutralizer 606 upon ATP 612 addition. The presence of the neutralizer 606 strongly reduces the charge at the sensor 604 surface, which would be restored by the displacement of the neutralizer 610 by a target molecule.

In FIG. 6B, DPV graph 620 shows signals obtained at the sensors before neutralization 622, after neutralization 624, and after ATP introduction 626. For ATP detection, sensors were incubated with a phosphate buffer solution (25 mM) containing 25 mM NaCl and different concentrations of ATP for 10 minutes at room temperature. Scans of uncomplexed immobilized aptamers revealed high catalytic current, consistent with the strong electrostatic attraction of Ru(III) to the DNA backbone of the aptamer. The observed current is reduced by > 80% when the neutralizer molecule is hybridized to the aptamer. Surprisingly, the reduction peak also shifts to more negative potentials. This may be due to slowing the kinetics of electron transfer when the neutralizer is present. When the analyte ATP binds to the aptamer probe a structural change of the aptamer causes release of the neutralizer, leading to an increase in catalytic current. As shown in concentration graph 630 of FIG. 6C, the observed change in current before ATP binding and after ATP binding was directly related to the concentration of ATP in solution. The horizontal dashed lines 632 show the signal generated in the absence of ATP.

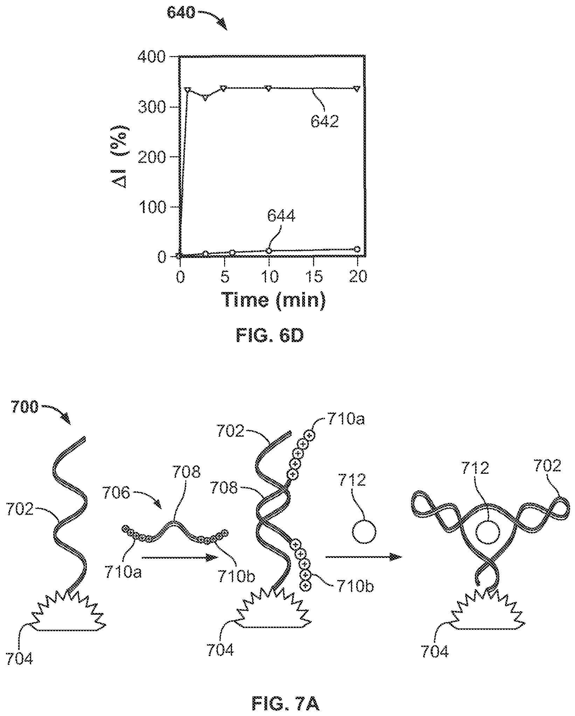

To evaluate the time dependence of the sensor response in certain embodiments, ATP was introduced into the [Ru(NH₃)₆]³⁺/[Fe(CN)₆]³⁻ catalytic solution and signal changes were measured in real time. FIG. 6D shows illustrative time graph 640, which contains data that indicate that signal changes in the presence 642 of ATP occur within 1 min, and the signal change in absence 644 of ATP is not significant even after 20 min. These results clearly indicate that sensor response is rapid, and that the probe-neutralizer complex is stable.

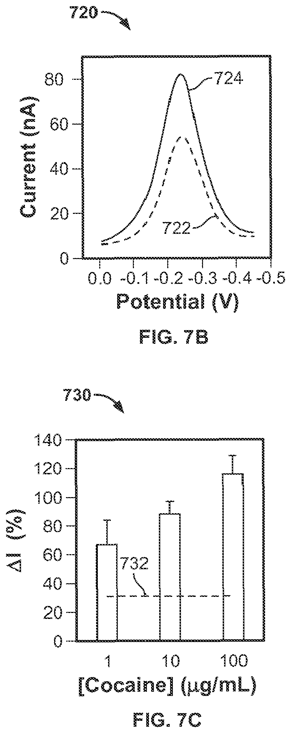

FIG. 7A shows an illustrative embodiment of a cocaine assay 700 that was carried out using a similar approach as described above for ATP, with aptamers 702 that were immobilized onto sensors 704 and specific for a cocaine molecule 712. DPV graph 720 of FIG. 7B shows the initial high current of the cocaine-binding aptamer decreased after neutralizer hybridization 722. Illustrative sequences for the aptamer 702 and neutralizer 706 used in the cocaine assay 700 are shown in FIG. 5. In this embodiment, the neutralizer 706 has a portion 708 that is complementary to the aptamer 702, and two mismatches 710 that permit facile release of the neutralizer 706 upon cocaine 712 addition. When the aptamer-neutralizer complex was challenged with cocaine 712, the resulting structural change of the aptamer 702 released the neutralizer 706 and resulted in a high catalytic current 724. For cocaine detection in this illustrative embodiment, sensors 704 were incubated with a phosphate buffer solution (25 mM) containing 25 mM NaCl and different concentrations of cocaine for 2 min at room temperature. Concentration graph 730 of FIG. 7C shows the observed change in current (in percentage points) on the y-axis against cocaine concentration (in µg/mL) on the x-axis. The horizontal dashed lines 732 show the signal generated in the absence of cocaine. The observed change in current before cocaine binding and after cocaine binding was directly related to the concentration of cocaine in solution. The signal change for 1 µg/mL cocaine was > 60% higher than that in the absence of cocaine or in the presence of a non-target analyte. This level of sensitivity is comparable with commercial tests and is ample for drug screening.

Having established a high level of performance with small molecule analytes, assay performance was evaluated to determine if it provided clinically-relevant (femtomolar or better) sensitivity against nucleic acid analytes, as other attempts to develop universal detection systems have not been successful in achieving good sensitivity with this analyte class.

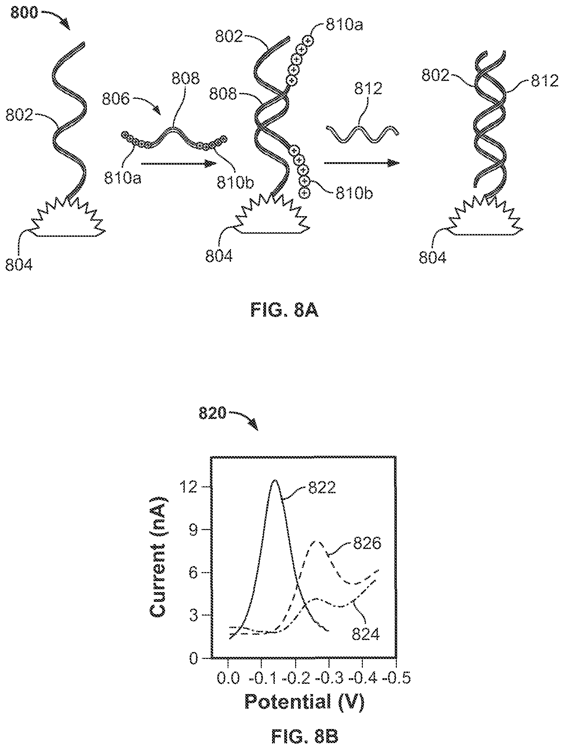

FIG. 8A illustrates a schematic representation 800 of an exemplary assay applied toward nucleic acids targets 812. A thiolated-DNA probe 802 was immobilized on the sensor 804. The catalytic current 822 for the uncomplexed DNA probe 802 was initially high, and was significantly suppressed 824 upon addition of neutralizer 806, as shown in DPV graph 820 of FIG. 8B. After exposure to 1 pM complementary oligonucleotide 20-mer target the current 826 increased by >300%. Sequences for the DNA probe 802, DNA probe neutralizer 806, and DNA target 812 are shown in FIG. 5. The neutralizer 806 has a portion 808 that is complementary to the DNA probe 802, and two mismatches 810 that permit facile release of the neutralizer 806 upon DNA target 812 addition.

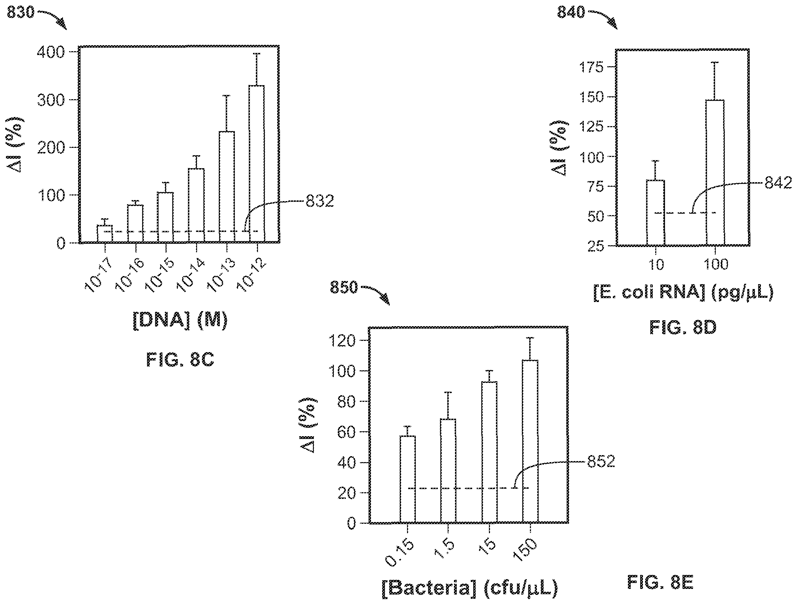

In certain embodiments, concentration dependence of a nucleic acid assay was studied using the 20-mer synthetic target DNA and a noncomplementary target, which was used to evaluate background levels and evaluate specificity. An illustrative sequence for the noncomplementary target is shown in FIG. 5. For this synthetic target DNA, sensors were incubated with a phosphate buffer solution (25 mM) containing 25 mM NaCl, 10 mM MgCl₂, and different concentrations of target for 30 minutes at room temperature. To identify the detection limit of the exemplary assay, the concentration of target DNA varied between 10pM and 10 aM, as shown in concentration graph 830 of FIG. 8C. Signal increased with increasing concentration of target within a range spanning 5 orders of magnitude. The horizontal dashed line 832 indicates the average currents of 100 nM the noncomplementary target. The signal change for 10 aM of DNA target, while above that of the 100 nM of the noncomplementary target, was not high enough to be statistically significant, indicating that the detection limit of this assay for 20-mer target is between 100 and 10 aM.

Performance of certain embodiments of assay with complex heterogeneous samples in the form *of E. coli* total RNA was also evaluated. The DNA probe was designed for RNA polymerase *β* mRNA (*rpoB*), a transcript that is highly expressed in bacteria and is not conserved between species. Illustrative sequences for the DNA probe and the neutralizer are shown in FIG. 5. As shown in the concentration graph 840 of FIG. 8D, concentration dependence of the signal produced by this illustrative assay on the concentration of a heterogeneous mixture *E. coli* total RNA, with the horizontal dashed line 842 showing the signal observed in the absence *of E. coli* RNA. In the cases *of E. coli* total RNA sensors were incubated with sterile and RNase-free PBS containing different concentrations of target for 30 minutes at room temperature. 10 pg/µL *E. coli* total RNA was successfully detected, indicating that the illustrative assay can achieve high levels of sensitivity with an excess of non-complementary material.

Certain embodiments of the assay that use unprocessed bacterial lysates are also provided and tested. In testing these embodiments, unprocessed bacterial lysates were generated by placing suspensions containing known quantities *of E. coli* into a lysis chamber, where strong electrical fields lysed the bacteria. This lysate was then used without further purification or amplification. Illustrative sequences used for the bacterial lysate probe and the bacterial lysate neutralizer are shown in FIG. 5. The concentration graph 850 of FIG. 8E illustrates the dependence of the assay signal on the quantity *of E. coli* used to produce the unprocessed lysates, and shows that signal increased with increasing concentration *of E. coli* bacteria in the initial suspensions. In these studies sensors were incubated with *E.coli* lysate in sterile and RNase-free PBS for 30 minutes at room temperature. The horizontal dashed line 852 in FIG. 8E represents average signal for lysate from 150 cfu/µL *Staphylococcus saprophyticus*, the components of which were noncomplementary to the probe. The detection limit for *E. coli* bacteria was, surprisingly, 0.15 cfu/µL. This sensitivity detection limit is unprecedented in direct analysis on unprocessed samples, and is clinically relevant for the detection of bacteria found in clinical samples. The kinetics of the assay in these illustrative embodiments allowed rapid detection of the presence of bacteria with high sensitivity and specificity, requiring less than 30 minutes from sample acquisition to result.

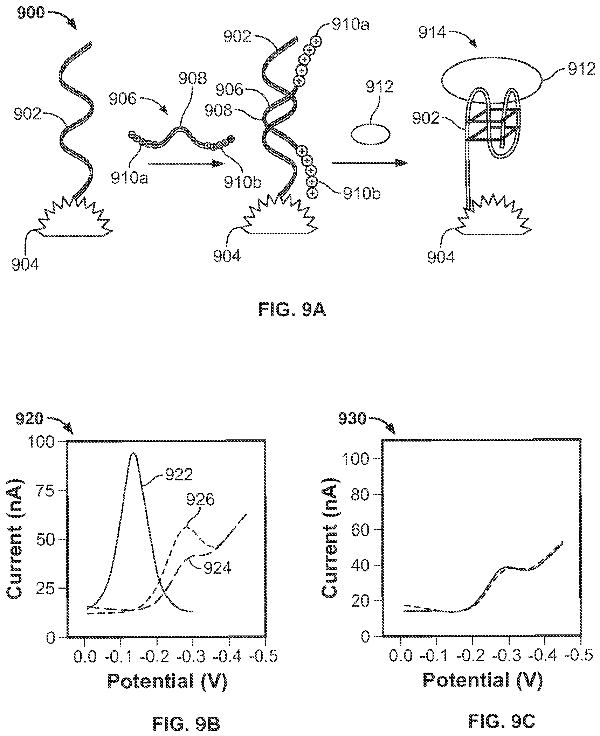

Certain embodiments were characterized to verify that the assay format described herein in connection with various embodiments could detect protein biomarkers, using thrombin as a model system. The thrombin binding aptamer is a well-characterized sequence that is known to fold into a G-quartet structure and bind thrombin at exosite I. Illustrative sequences for the thrombin binding aptamer and the thrombin aptamer neutralizer are shown in FIG. 5. FIG. 9A shows an illustrative protein detection method 900 according to certain embodiments. A thiolated thrombin-binding aptamer 902 was deposited onto a sensor surface 904 and subsequently complexed with thrombin aptamer neutralizer 906, which at least partially neutralizes the charge near the sensor surface 904. The neutralizer 906 has a portion 908 that is complementary to the aptamer 902, and two mismatches 910 that permit facile release of the neutralizer 906 upon thrombin 912 addition. Binding of thrombin 912 to the thrombin binding aptamer 902, which forms an aptamer-thrombin complex 914, displaces the thrombin aptamer neutralizer 906 and restores the charge, resulting in a detectable change in sensor response.

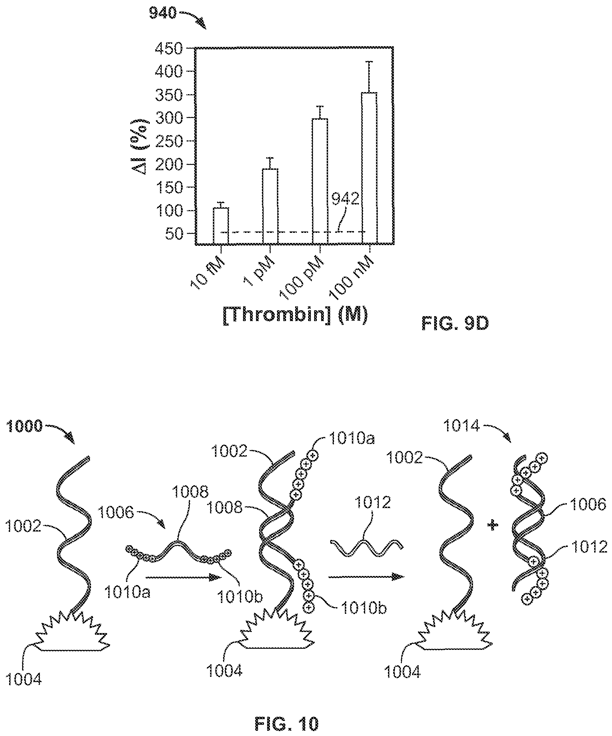

In FIG. 9B, DPV graph 920 shows electrocatalytic currents of the aptamer alone 922, the aptamer-neutralizer complex 924, and the aptamer-thrombin complex 926. The electrocatalytic current was clearly suppressed upon neutralizer binding to the thrombin aptamer. When treated with 100 pM thrombin, a large increase in catalytic current was observed. Conversely, DPV graph 930 of FIG. 9C shows that the signal change was negligible when treated with 100 nM BSA (a nonspecific protein), indicating that the assay was specific for thrombin. As shown in concentration graph 940 of FIG. 9D, the observed signal was directly proportional to thrombin concentration, and showed that as low as 10 fM thrombin was clearly detectable. The horizontal dashed lines 942 show the signal generated in the absence of thrombin.

FIG. 10 shows an illustrative embodiment of a nucleic acid assay 1000 in which the analyte displaces the pseudoligand by forming a complex with the pseudoligand rather than the probe. In this embodiment, the neutralizer 1006 has a greater affinity for the analyte 1012 than it does for the probe 1002, which results in the formation of a complex 1014 between the analyte 1012 and the neutralizer 1006. In assay 1000, the neutralizer 1006 is specific for the analyte 1012 and incorporates one or more base pair mismatches 1010 with the probe 1002, and a portion 1008 that is complementary to the probe 1002. In this embodiment, the signal observed after the neutralizer 1006 hybridizes to the probe 1002 is increased by the removal of the mismatched neutralizer 1006 from the probe 1002 due to hybridization with a perfectly-matched target 1012 that is in solution. This leads to charge restoration at the electrode detection surface 1004 in the absence of an analyte-probe complex, based on removal of the neutralizer 1006 and restoration of the charge of the probe 1002 that is affixed to the electrode detection surface 1004. It is to be understood that, although this embodiment is described in the context of nucleic acid detection, the embodiment is not so limited, and may be used to detect a wide variety of analytes.

Thus, specific embodiments and applications of a sensitive biosensor applicable to a wide range of biological molecules have been disclosed. It should be apparent, however, to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "includes", "including", "contains", "containing", "has", "have", having", "comprises" and "comprising," as used herein, should be interpreted as referring to elements, components, or steps in a non-exclusive manner, indicating that the referenced elements, components, or steps may be present, or utilized, or combined with other elements, components, or steps that are not expressly referenced. The term "plurality," as used herein means more than one, and may include any defined or undefined subset of two or more steps, elements, or components. Furthermore, where a definition or use of a term in a reference, which is incorporated by reference herein, is inconsistent or contrary to the definition of that term provided herein, the definition of that term provided herein applies and the definition of that term in the reference does not apply.

The application of which this description and claims form part may be used as a basis for priority in respect of any subsequent application. The claims of such subsequent application may be directed to any feature or combination of features described herein. They may take the form of product, method or use claims and may include, by way of example and without limitation, one or more of the following claims.

The present disclosure discloses at least the following items:
Item 1. A method of detecting an analyte, comprising;
   contacting a sample with a reversible first complex comprising a probe affixed to a sensor surface and a pseudoligand having a charge opposed to that of the probe; and
   detecting the presence of a second complex formed by displacement of the pseudoligand from the probe by the analyte, if present in the sample, wherein the presence of the second complex indicates the presence of the analyte.
Item 2. The method of item 1, wherein the first complex has a first charge state and the second complex has a second charge state, and wherein detecting the presence of the second complex comprises determining a difference between the first charge state and the second charge state.
Item 3. The method of item 1 or 2, wherein the second charge state has a greater overall magnitude than the first charge state.
Item 4. The method of any of items 1-3, wherein detecting the presence of the second complex comprises measuring a change in current amplitude caused by for the formation of the second complex.
Item 5. The method of item 4, wherein an increase in the current amplitude above a predetermined threshold is indicative of the presence of the analyte.
Item 6. The method of items 4 or 5, wherein the magnitude of the change in the current amplitude is indicative of the concentration of the analyte in the sample.
Item 7. The method of any of items 1-3, wherein detecting comprises measuring a change in voltage caused by the formation of the second complex.
Item 8. The method of any of items 1-7, wherein an affinity of the probe for the pseudoligand is less than an affinity of the probe for the analyte.
Item 9. The method of any of items 1-7, wherein an affinity of the probe for the pseudoligand is greater than an affinity of the probe for the analyte.
Item 10. The method of any of items 1-9, wherein the relative stabilities of the first complex and the second complex are modified by manipulating temperature.
Item 11. The method of any of items 1-10, wherein the relative stabilities of the first complex and the second complex are modified by the composition of the buffer.
Item 12. The method of any of items 1-11, wherein the analyte is a nucleic acid.
Item 13. The method of any of items 1-11, wherein the analyte is a protein or protein fragment.
Item 14. The method of items 1-11, wherein the analyte is selected from the group consisting of nucleotides, nucleotide analogs, drugs of abuse, therapeutic drugs, and environmental contaminants.
Item 15. The method of any of items 1-11, wherein the analyte has a molecular weight of less than about 500 daltons.
Item 16. The method of any of items 1-15, wherein the probe comprises a nucleic acid.
Item 17. The method of any of items 1-16, wherein the pseudoligand comprises a PNA.
Item 18. The method of item 17, wherein the PNA comprises one or more appended cationic functional groups.
Item 19. The method of items 17 or 18, wherein the PNA comprises one or more base pair mismatches with a probe nucleic acid sequence.
Item 20. The method of item 1, further comprising forming the second complex between the pseudoligand and the analyte.
Item 21. The method of item 1, further comprising forming the second complex between the probe and the analyte.
Item 22. A device for detecting an analyte, comprising:
   a sensor having a sensor surface, the sensor surface having a probe affixed thereto;
   an inlet for contacting a sample with a reversible first complex comprising the probe and a pseudoligand having a charge opposed to that of the probe; and
   a detection unit for detecting the presence of a second complex formed by displacement of the pseudoligand from the probe by the analyte, if present in the sample, wherein the presence of the second complex indicates the presence of the analyte.
Item 23. The device of item 22, wherein detecting the presence of a second complex comprises detecting an increase in charge at or near the sensor surface.
Item 24. The device of items 22 or 23, further comprising a plurality of sensor surfaces wherein at least two of the sensor surfaces comprise different probes.
Item 25. The device of items 22-24, wherein the first complex is formed prior to contacting the sample with the first complex.
Item 26. The device of any of items 22-25, wherein the sensor is configured to provide a response that is dependent on the charge at said sensor surface.
Item 27. The device of any of items 22-26, wherein the sensor is selected from the group consisting of a nanostructured electrochemical detection electrode, a field-effect transistor, a microcantilever, and an electrochemical sensor.
Item 28. The device of any of items 22-27, wherein the analyte is a nucleic acid.
Item 29. The device of any of items 22-2'I, wherein the analyte is a protein or protein fragment.
Item 30. The device of any of items 22-27, wherein the analyte has a molecular weight of less than about 500 daltons.
Item 31. The device of any of items 22-30, wherein the probe comprises a nucleic acid.
Item 32. The device of any of items 22-31 , wherein the pseudoligand comprises a PNA.
Item 33. The device of item 32, wherein the PNA comprises appended cationic functional groups.
Item 34. The device of items 32 or 33, wherein the PNA comprises one or more base pair mismatches with a probe nucleic acid sequence.
Item 35. A system for detecting an analyte, comprising:
   a sensor having a sensor surface, the sensor surface having a probe affixed thereto;
   an inlet for contacting a sample with a reversible first complex comprising the probe and a pseudoligand having a charge opposed to that of the probe;
   a detection unit for detecting the presence of a second complex formed by the analyte, if present in the sample, and at least one of the pseudoligand or the probe, wherein the presence of the second complex indicates the presence of the analyte; and
   a communication unit in communication with the detection unit for communicating a result of the detection to a user.
Item 36. The system of item 35, wherein the detection unit is configured to detect the presence of a second complex by measuring a difference in charge at the sensor surface.
Item 37. The system of items 35 or 36, further comprising a plurality of sensor surfaces wherein at least two of the sensor surfaces comprise different probes.
Item 38. The system of items 35-37, wherein the first complex is formed prior to contacting the sample with the first complex.
Item 39. The system of any of items 35-38, wherein the sensor is configured to provide a response that is dependent on the charge at said sensor surface.
Item 40. The system of any of items 35-39, wherein the sensor is selected from the group consisting of a nanostructured electrochemical detection electrode, a field-effect transistor, a microcantilever, and an electrochemical sensor.
Item 41. The system of any of items 35-40, wherein the analyte is a nucleic acid.
Item 42. The system of any of items 35-40, wherein the analyte is a protein or protein fragment.
Item 43. The system of any of items 35-40, wherein the analyte has a molecular weight of less than about 500 daltons.
Item 44. The system of any of items 35-43, wherein the probe comprises a nucleic acid.
Item 45. The system of any of items 35-44, wherein the pseudoligand comprises a PNA.
Item 46. The system of item 45, wherein the PNA comprises appended cationic functional groups.
Item 47. The system of items 45 or 46, wherein the PNA comprises one or more base pair mismatches with a probe nucleic acid sequence.
Item 48. An assay composition for use in drug screening, comprising a reversible first complex comprising a probe molecule affixed to a sensor surface and a pseudoligand having complementarity with said probe molecule, said first complex having a first charge state, wherein an affinity of the probe for the pseudoligand is less than an affinity of the probe for the drug to thereby allow said drug to displace the pseudoligand from the first complex to form a second complex and wherein the second complex has a second charge state.
Item 49. The assay composition of item 48, wherein the second charge state has a greater overall magnitude than the first charge state.
Item 50. The assay composition of items 48 or 49, wherein the probe comprises a nucleic acid.
Item 51. The assay composition of any of items 48-50, wherein the pseudoligand comprises a PNA.
Item 52. The assay composition of item 51 , wherein the PNA comprises one or more appended cationic functional groups.
Item 53. The assay composition of items 51 or 52, wherein the PNA comprises one or more base pair mismatches with a probe nucleic acid sequence.
Item 54. An assay composition of any of items 48-53, wherein the drug is a therapeutic drug.
Item 55. An assay composition of any of items 48-53, wherein the drug is a drug of abuse.
Item 56. A kit comprising a device having a sensor surface with a probe affixed thereto, wherein the kit further comprises a pseudoligand capable of forming a reversible complex with the probe and having a charge opposed to that of the probe.
Item 57. The kit of item 56, further comprising a detection unit for detecting a difference in charge at the sensor surface.
Item 58. The kit of items 56 or 57, wherein the first complex is formed prior to contacting the sample with the first complex.
Item 59. The kit of any of items 56-58, wherein the sensor is configured to provide a response that is dependent on the charge at said sensor surface.
Item 60. The kit of any of items 56-59, wherein the sensor is selected from the group consisting of a nanostructured electrochemical detection electrode, a field-effect transistor, a microcantilever, and an electrochemical sensor.
Item 61. The kit of any of items 56-60, wherein the analyte is a nucleic acid.
Item 62. The kit of any of items 56-60, wherein the analyte is a protein or protein fragment.
Item 63. The kit of any of items 56-60, wherein the analyte has a molecular weight of less than about 500 daltons.
Item 64. The kit of any of items 56-63, wherein the probe comprises a nucleic acid.
Item 65. The kit of any of items 56-64, wherein the pseudoligand comprises a PNA.
Item 66. The kit of item 65, wherein the PNA comprises appended cationic functional groups.
Item 67. The kit of items 65 or 66, wherein the PNA comprises one or more base pair mismatches with a probe nucleic acid sequence.
Item 68. A sensor for detecting an analyte comprising:
   a sensor surface comprising a plurality of electrodes;
   a probe affixed to at least one of the plurality of electrodes; and
   a pseudoligand capable of forming a reversible first complex with the probe such that the pseudoligand is displaced by an analyte when contacted with the first complex.
Item 69. The sensor of item 68, wherein a second complex is formed when the pseudoligand is displaced.
Item 70. The sensor of item 69, further comprising a detection unit that is configured to detect the presence of a second complex by measuring a difference in charge at the sensor surface.
Item 71. The sensor of items 68-70, wherein different probes are affixed to each of the the plurality of electrodes.
Item 72. The sensor of items 68-71, wherein the first complex is formed prior to contact with a sample containing the analyte.
Item 73. The sensor of any of items 68-72, wherein the sensor is configured to provide a response that is dependent on the charge at said sensor surface.
Item 74. The sensor of any of items 68-73, wherein the sensor is selected from the group consisting of a nanostructured electrochemical detection electrode, a field-effect transistor, a microcantilever, and an electrochemical sensor.
Item 75. The sensor of any of items 68-74, wherein the analyte is a nucleic acid.
Item 76. The sensor of any of items 68-74, wherein the analyte is a protein or protein fragment.
Item 77. The sensor of any of items 68-74, wherein the analyte has a molecular weight of less than about 500 daltons.
Item 78. The sensor of any of items 68-77, wherein the probe comprises a nucleic acid.
Item 79. The sensor of any of items 68-77, wherein the pseudoligand comprises a PNA.
Item 80. The sensor of item 79, wherein the PNA comprises appended cationic functional groups.
Item 81. The sensor of items 79 or 80, wherein the PNA comprises one or more base pair mismatches with a probe nucleic acid sequence.

## Claims

1. A device for detecting an analyte, comprising:
a sensor having a sensor surface, the sensor surface having a probe affixed thereto;
an inlet for contacting a sample with a reversible first complex comprising the probe and a pseudoligand having a charge opposed to that of the probe; and
a detection unit for detecting the presence of a second complex formed by displacement of the pseudoligand from the probe by the analyte, the detection unit being configured to measure a change in charge at or near the sensor surface.

2. The device of claim 1, wherein detecting the presence of a second complex comprises detecting an increase in charge at or near the sensor surface.

3. The device of claim 1 or 2, wherein the sensor surface is one of a plurality of sensor surfaces wherein at least two of the plurality of sensor surfaces have different probes affixed thereto.

4. The device of any of claims 1-3, wherein the sensor is configured to provide a response that is dependent on the charge at the sensor surface.

5. The device of any of claims 1-4, wherein the sensor is selected from the group consisting of a nanostructured electrochemical detection electrode, a field-effect transistor, a microcantilever, and an electrochemical sensor.

6. The device of any of claims 1-5, wherein the probe comprises a nucleic acid.

7. The device of any of claims 1-6, wherein the pseudoligand comprises a PNA.

8. The device of claim 7, wherein the PNA comprises appended cationic functional groups.

9. The device of claim 7 or 8, wherein the PNA comprises one or more base pair mismatches with a probe nucleic acid sequence.

10. The device of any of claims 1-9, wherein the second complex is formed between the pseudoligand and the analyte or between the probe and the analyte.

11. The device of any of claims 1-9, wherein an affinity of the probe for the pseudoligand is less than an affinity of the probe for the analyte.

12. The device of any of claims 1-9, wherein an affinity of the pseudoligand for the analyte is grater than an affinity of the probe for the analyte.

13. The device of any of claims 1-12, comprising means for modifying the relative stabilities of the first complex and the second complex by manipulating temperature.

14. A system for detecting an analyte, comprising:
a sensor according to any of claims 1-13; and
a communication unit in communication with the detection unit for communicating a result of the detection to a user.

15. An assay composition for use in a device according to any of claims 1-13 in drug screening, comprising the reversible first complex, the pseudoligand having complementarity with the probe molecule, wherein the first complex has a first charge state and the second complex has a second charge state.
